# EUROPEAN PATENT SPECIFICATION

(11) **EP 3 556 406 B1**
(45) Date of publication and mention of the grant of the patent: **22.12.2021**
(21) Application number: 19174604.9
(22) Date of filing: 02.05.2014
(51) Int. Cl.: A61L 27/00

(54) **METHOD FOR PRODUCING PARTICULATE DECELLULARIZED TISSUE**
VERFAHREN ZUR HERSTELLUNG EINES PARTIKELFÖRMIGEN DEZELLULARISIERTEN GEWEBES
PROCÉDÉ DE PRODUCTION D'UN TISSU DÉCELLULARISÉ PARTICULAIRE

(30) Priority: 07.05.2013 JP 2013097402
(43) Date of publication of application: 23.10.2019
(62) Divisional of application: 14794303.9
(73) Proprietor: National University Corporation Tokyo Medical and Dental University, Bunkyo-ku Tokyo 113-8510 (JP); Adeka Corporation, Tokyo 116-8554 (JP)
(72) Inventor: KISHIDA, Akio, Tokyo, 113-8510 (JP); KIMURA, Tsuyoshi, Tokyo, 113-8510 (JP); NEGISHI, Jun, Tokyo, 116-8554 (JP); HIWATARI, Ken-ichiro, Tokyo, 116-8554 (JP); TASAKI, Akiko, Tokyo, 116-8554 (JP)
(74) Representative: Vossius & Partner Patentanwälte Rechtsanwälte mbB

(56) References cited:
- EP-A2- 2 345 435
- US-A1- 2011 044 847
- HASHIMOTO Y ET AL: "The effect of decellularized bone/bone marrow produced by high-hydrostatic pressurization on the osteogenic differentiation of mesenchymal stem cells", BIOMATERIALS, ELSEVIER SCIENCE PUBLISHERS BV., BARKING, GB, vol. 32, no. 29, 1 October 2011 (2011-10-01), pages 7060-7067, XP002711760, ISSN: 0142-9612, DOI: 10.1016/J.BIOMATERIALS.2011.06.008 [retrieved on 2011-07-02]
- JUN NEGISHI ET AL: "Effect of treatment temperature on collagen structures of the decellularized carotid artery using high hydrostatic pressure", JOURNAL OF ARTIFICIAL ORGANS ; THE OFFICIAL JOURNAL OF THE JAPANESE SOCIETY FOR ARTIFICIAL ORGANS, SPRINGER-VERLAG, TO, vol. 14, no. 3, 11 May 2011 (2011-05-11), pages 223-231, XP019948540, ISSN: 1619-0904, DOI: 10.1007/S10047-011-0570-Z
- ISARAWUT PRASERTSUNG ET AL: "Development of acellular dermis from porcine skin using periodic pressurized technique", JOURNAL OF BIOMEDICAL MATERIALS RESEARCH. PART B: APPLIED BIOMATERIALS, JOHN WILEY & SONS, HOBOKEN, NJ, US, vol. 85, no. 1, 1 April 2008 (2008-04-01), pages 210-219, XP002670156, ISSN: 1552-4973, DOI: 10.1002/JBM.B.30938 [retrieved on 2007-09-12]
- "ACELLULAR MATRICES FOR THE TREATMENT OF WOUNDS an expert working group review", , 31 December 2010 (2010-12-31), XP055253451, Retrieved from the Internet: URL:http://www.woundsinternational.com/med ia/issues/380/files/content_9732.pdf [retrieved on 2016-02-26]

## Description

The present invention relates to a particulate decellularized tissue for use in therapy or for use in surgery

### BACKGROUND ART

When a graft is transplanted from biological tissues of others, rejection by the tissue of the subject who received the graft becomes a problem. For solving such a problem, development of a prosthesis is expected. As material, a variety of macromolecules have been attempted. However, since compatibility between such material and biological tissues is low, a graft may be dropped out from the joining site or infectious diseases may occur. Thus, in order to improve compatibility with biological tissues, the technique has been developed to use decellularized biological tissues, which are supporting tissues that remain after removal of cells from biological tissues, as a graft. For decellularization of biological tissues, known methods are the use of a surfactant (e.g. cf. Patent references 1 and 2), the use of an enzyme (e.g. cf. Patent reference 3), the use of an acidifying agent (e.g. cf. Patent reference 4), treatment with a super-high hydrostatic pressure (e.g. cf. Patent references 5 to 7), and the like.

A decellularized tissue in a granulated or pulverized form (particulate decellularized tissue) is also known. A particulate decellularized tissue has been used for prompting regeneration and cure of the affected sites through its injection thereto or molded for use as a graft (e.g. cf. Patent references 8 to 10). A hitherto known particulate decellularized tissue has been prepared by using a surfactant. However, a particulate decellularized tissue prepared by treatment with a super-high hydrostatic pressure is not known.
Patent reference 1: Japanese Patent Publication No. 60-501540
Patent reference 2: Japanese Patent Publication No. 2003-518981
Patent reference 3: Japanese Patent Publication No. 2002-507907
Patent reference 4: Japanese Patent Publication No. 2003-525062
Patent reference 5: Japanese Patent Publication No. 2004-094552
Patent reference 6: WO 2008/111530
Patent reference 7: Japanese Patent Publication No. 2013-502275
Patent reference 8: Japanese Patent Publication No. 07-509638
Patent reference 9: Japanese Patent Publication No. 2002-518319
Patent reference 10: Japanese Patent Publication No. 2012-505013 (EP2345435)

### DISCLOSURE OF THE INVENTION

### (Technical Problem to be Solved by the Invention)

A hitherto known particulate decellularized tissue does not cause graft rejection and is useful for regeneration and cure of the affected sites. However, there were problems that it does not show sufficiently effective tissue regeneration and shows cytotoxicity when used as material for cell culture.

### (Means for Solving the Problems)

The present inventors have earnestly studied to solve the above problems and as a result have found that particulate animal tissues which are decellularized by applying a high hydrostatic pressure in a medium show the effect of cellular attraction and the effect of induction of cellular differentiation but do not show cytotoxicity while cell culture to thereby complete the present invention. Thus, the present invention relates to a particulate decellularized tissue for use in therapy or for use in surgery, wherein said particulate decellularized tissue is prepared by a method for preparing a particulate decellularized tissue comprising a step of applying a high hydrostatic pressure to animal-derived tissues in a medium, wherein said high hydrostatic pressure is 2 to 1,500 MPa, and wherein said high hydrostatic pressure is applied after the animal-derived tissues are pulverized.

The invention also relates to this particulate decellularized tissue for these uses wherein the method by which the particulate decellularized tissue is prepared comprises that the animal-derived decellularized tissues obtained by applying a high hydrostatic pressure to the animal-derived tissues in a medium are washed with a washing liquid preferably a washing liquid not comprising an anionic surfactant and/or a nonionic surfactant. The invention also relates to this particulate decellularized tissue for these uses wherein the particulate decellularized tissue is a particulate dcellularized tissue for use in treating affected tissue, preferably for treating a wound or frostbite.

### EFFECTS OF THE INVENTION

In accordance with the method, a particulate decellularized tissue is obtained which does not show cytotoxicity but shows the effect of cellular attraction and the effect of induction of cellular differentiation and shows a high effect of tissue regeneration. A particulate decellularized tissue obtained by the method, due to its particulate form, is not limited for its applicable sites but can be applied to a variety of sites.

### BRIEF DESCRIPTION OF DRAWINGS

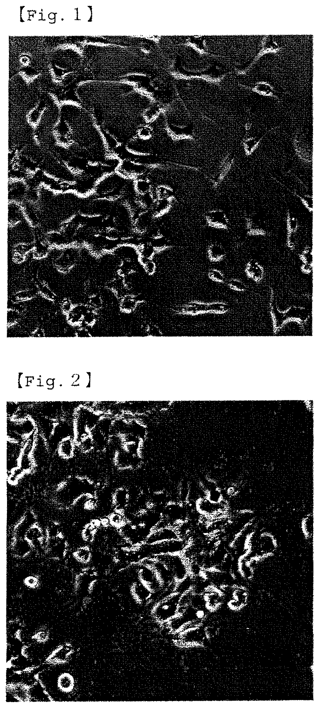

Fig. 1 shows the results of the neurite outgrowth test in Example 2 without addition of NGF.
Fig. 2 shows the results of the neurite outgrowth test in Example 3 without addition of NGF.

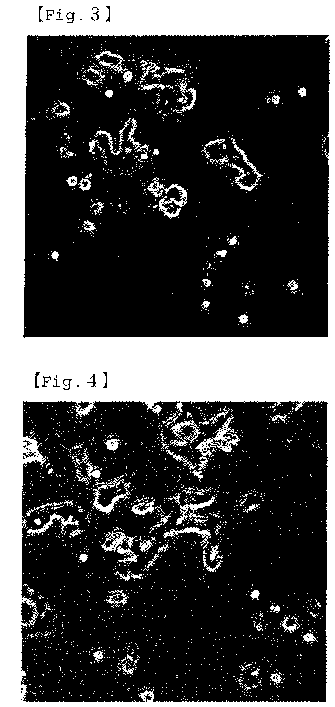

Fig. 3 shows the results of the neurite outgrowth test for blank without addition of NGF.
Fig. 4 shows the results of the neurite outgrowth test for blank with addition of NGF.

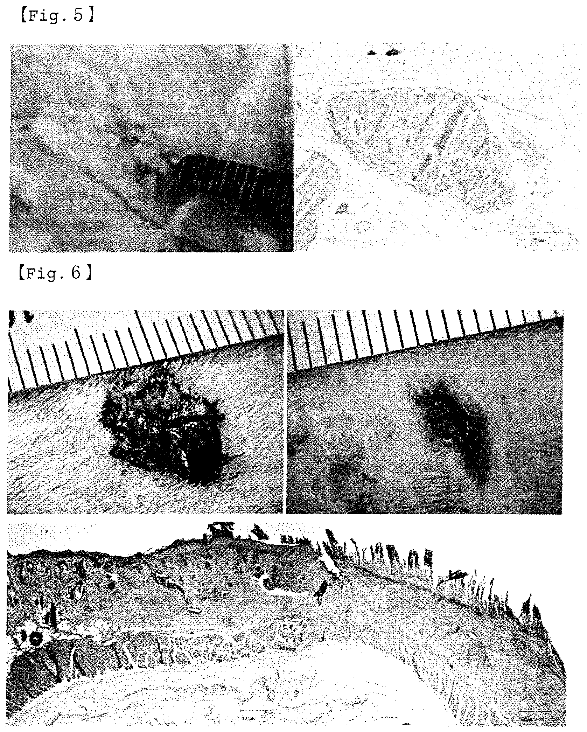

Fig. 5 shows the subcutaneous pocket (left) and the section of the stained rat tissue (right) on Day 28 in the subcutaneous enthesis test in Example 1.
Fig. 6 shows the appearance before test (upper left), the appearance after test (upper right) and the section of the stained rat tissue (lower) in the frostbite model test in Example 1.

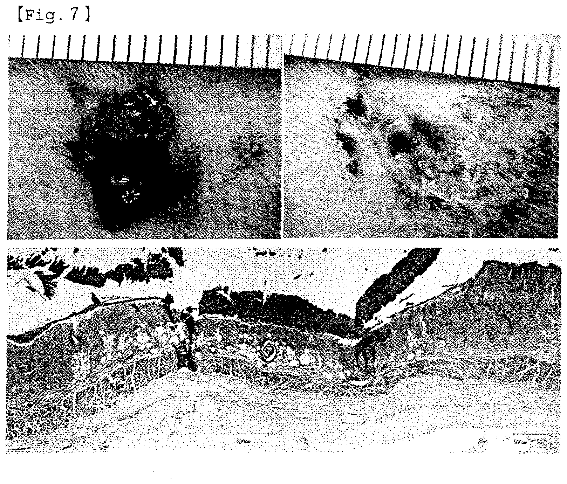

Fig. 7 shows the appearance before test (upper left), the appearance after test (upper right) and the section of the stained rat tissue (lower) for blank in the frostbite model test.

### BEST MODE FOR CARRYING OUT THE INVENTION

The present invention is explained in detail in the following.

### Biological tissue

Biological tissues for use in the method for preparing a particulate decellularized tissue of the present invention are not particularly limited insofar as they comprise cells from vertebrate animals but preferably are those from mammals or birds in view of low rejection and more preferably are those from domestic animals of mammals or domestic animals of birds or human in view of availability. The domestic animals of mammals include cattle, horses, camels, llama, donkey, yak, sheep, pigs, goats, deer, alpacas, dogs, raccoon dogs, weasels, foxes, cats, rabbits, hamsters, guinea pigs, rats, mice, squirrels, raccoons, and the like. The domestic animals of birds include parakeet, parrot, chicken, ducks, turkeys, geese, guinea fowl, pheasant, ostrich, quail, and the like. Among these, biological tissues from pigs, rabbits and human are preferable in view of stable availability.

The body parts from which biological tissues are obtained may be those which have an extracellular matrix structure, including liver, kidney, ureter, bladder, urethra, tongue, tonsils, esophagus, stomach, small intestine, large intestine, anus, pancreas, heart, blood vessels, spleen, lung, brain, bone, spinal cord, cartilage, testis, uterus, fallopian tube, ovary, placenta, cornea, skeletal muscle, tendons, nerves, skin, and the like. The body parts from which biological tissues are obtained are preferably cartilage, bone, liver, kidney, heart, lung, brain and spinal cord in view of their high tissue regeneration. The biological tissues, after removal, are preferably treated for preventing putrefaction or decrease in the function, including germicidal treatment with chemicals, freezing treatment by refrigeration, and the like, preferably freezing treatment in view of low damage to the tissues.

### Pulverizing step

In the method for preparing a particulate decellularized tissue of the present invention, biological tissues (or decellularized tissues) are pulverized into particulates after removal of biological tissues, and before application of a high hydrostatic pressure to the biological tissues, washing and removal of the destroyed cells, to obtention of particulate decellularized tissue. They are pulverized before washing and removal of the cells since the destroyed cells in a particulate form can be washed and removed more easily than they are in the biological tissues the shape of which is maintained.

A method for pulverizing the biological tissues includes, but is not limited to, pulverization of biological tissues as they are at an ordinary temperature, refrigeration of biological tissues and pulverization thereof under frozen conditions, and the like. However, in case of biological tissues which are difficult to be pulverized at an ordinary temperature such as soft tissues e.g. kidney, they are preferably pulverized under frozen conditions. In case of pulverization under frozen conditions, since tissues are sometimes damaged due to ice crystal growth at around 0°C, a pulverization temperature is preferably -80°C to -5°C, more preferably -50°C to -10°C, and most preferably -40°C to -15°C. Since biological tissues are preferably preserved under frozen conditions, pulverization of biological tissues preserved under frozen conditions would simplify the steps and allow for treatment of particulate biological tissues with a high hydrostatic pressure.

Biological tissues may also be pulverized after drying wherein classification after pulverization is easier. A drying method of biological tissues includes drying with heating, drying under reduced pressure, lyophilization, dehydration with an organic solvent, and the like, preferably lyophilization and dehydration with an organic solvent, more preferably lyophilization, in view of easier washing of cells and nucleic acids. An organic solvent for dehydration includes ethanol, acetone, and the like.

A method for pulverization includes ball mill, a bead mill, a colloid mill, a conical mill, disc mill, edge mills, milling mills, hammer mills, pellet mills, cutting mills, roller mills, jet mills, and the like, preferably cutting mills in view of lesser damage to biological tissues.

When a size of a particulate decellularized tissue of the present invention is too small, the effect of tissue regeneration is low. On the other hand, when a size of a particulate decellularized tissue of the present invention is too large, it becomes difficult to use the tissue as a material for transplant or treatment. Therefore, a particulate decellularized tissue of the present invention preferably has a particle size of 0.1 to 1,000 µm, more preferably 0.5 to 500 pm, and most preferably 1 to 100 µm.

### Step of high hydrostatic pressure treatment

In accordance with the method, a hydrostatic pressure is applied to biological tissues in a medium to decellularize the tissues. When a hydrostatic pressure is less than 100 MPa, decellularization from biological tissues is insufficient. On the other hand, application of a hydrostatic pressure requires a pressure container tolerable to the application of a pressure and needs immense energy. Thus, a hydrostatic pressure applied to biological tissues is preferably 2 to 1,500 MPa, more preferably 10 to 1,000 MPa, and most preferably 80 to 500 MPa.

A medium used when a hydrostatic pressure is applied includes water, saline, propylene glycol or an aqueous solution thereof, glycerol or an aqueous solution thereof, an aqueous solution of saccharides, and the like. A buffer includes acetate buffer, phosphate buffer, citrate buffer, borate buffer, tartrate buffer, Tris buffer, HEPES buffer, MES buffer, and the like. Saccharides in an aqueous solution of saccharides include erythrose, xylose, arabinose, allose, talose, glucose, mannose, galactose, erythritol, xylitol, mannitol, sorbitol, galactitol, sucrose, lactose, maltose, trehalose, dextran, alginic acid, hyaluronic acid, and the like.

A temperature at which high hydrostatic pressure treatment is carried out is not limited insofar as ice is not formed and there is no heat damage to tissues and is preferably 5 to 45°C, more preferably 10 to 40°C, and most preferably 15 to 35°C, in view of smooth treatment of decellularization and lesser impact on tissues. When a processing time of a high hydrostatic pressure is too short, cell disruption is not sufficient whereas a too long processing time of a high hydrostatic pressure leads to waste of energy. Therefore, a processing time of a high hydrostatic pressure is preferably 5 to 60 minutes, more preferably 7 to 30 minutes.

### Washing step

From biological tissues to which a high hydrostatic pressure is applied, destroyed cells are washed and removed with a washing liquid. A washing liquid may be the same as, or different from, that used when a hydrostatic pressure is applied or may be a combination of plural kinds. A washing liquid preferably contains a nuclease, an organic solvent or a chelating agent. A nuclease and an organic solvent may improve a removal rate of a nucleic acid constituent and a lipid from the biological tissues to which a hydrostatic pressure is applied, respectively, whereas a chelating agent may prevent calcification when a particulate decellularized tissue of the present invention is applied to the affected portions through inactivation of calcium ions and magnesium ions in the decellularized tissue.

An organic solvent is preferably an aqueous organic solvent in view of higher removal of a lipid, and is preferably ethanol, isopropanol, acetone and dimethyl sulfoxide. A chelating agent includes an iminocarboxylic acid chelating agent or a salt thereof such as ethylenediaminetetraacetic acid (EDTA), nitrilotriacetic acid (NTA), diethylene tri amine pentaacetic acid (DTPA), hydroxyethyl ethylenediamine triacetic acid (HEDTA), triethylenetetraminehexaacetic acid (TTHA), 1,3-propane diamine tetraacetic acid (PDTA), 1,3-diamino-2-hydroxypropane tetraacetic acid (DPTA-OH), hydroxyethyl-iminodiacetic acid (HIDA), dihydroxyethylglycine (DHEG), glycol ether diaminetetraacetic acid (GEDTA), dicarboxymethyl glutamic acid (CMGA), 3-hydroxy-2,2'-iminodisuccinic acid (HIDA), dicarboxymethyl aspartic acid (ASDA); and a hydroxycarboxylic acid chelating agent or a salt thereof such as citric acid, tartaric acid, malic acid and lactic acid. A salt of these chelating agents includes a sodium salt or potassium salt.

When a washing liquid contains an anionic surfactant or a nonionic surfactant, a removal rate of the destroyed cellular tissues, nucleic acids and lipids increases but cytotoxicity or a decrease in tissue regeneration of decellularized tissues are sometimes observed. Therefore, it is preferable not to use such a surfactant. For washing, particulate tissues obtained after a high hydrostatic pressure treatment are immersed in a washing liquid which may be shaken or stirred as needed.

The affected sites to which the particulate decellularized tissue of the present invention is applied are not particularly limited but may be any affected sites insofar as they comprise cellular tissues. The particulate decellularized tissue of the present invention may be applied to the same biological tissues as, or different biological tissues from, those from which the particulate decellularized tissue is derived. For instance, when the particulate decellularized tissue of the present invention is the one from the liver, it may be used to the liver or to the different sites such as the kidney, the heart, the lung, the brain and the spinal cord.

The particulate decellularized tissue of the present invention may be applied to the affected sites as it is, or as a dispersing liquid in a saline, 5% glucose solution, Ringer's solution, and the like, or as a gel with fibrinogen, and the like. The particulate decellularized tissue of the present invention may be applied alone or in combination with other ingredients effective for regeneration and cure of the affected sites. The other ingredients include growth factor, proteoglycan or glycosaminoglycan, cells, β-1,3-glucan, mevalonic acid, and the like.

Growth factor includes, insulin analogues growth factor (IGF), basic fibroblast growth factor (bFGF), acidic fibroblast growth factor (aFGF), transforming growth factor-α (TGF-α), transforming growth factor-β (TGF-β), bone morphogenetic protein (BMP), platelet derived growth factor (PDGF), keratinocyte growth factor (KGF), epidermal growth factor (EGF), vascular endothelial growth factor (VEGF), hematopoietic accelerating factor (EPO), granulocyte macrophage growth factor (GM-CSF), the condyle Fe d321 grain growth factor (G-CSF), nerve growth factor (NGF), heparin-binding factor (EGF), and the like. Proteoglycan or glycosaminoglycan includes chondroitin sulfate, heparan sulfate, keratan sulfate, dermatan sulfate, hyaluronic acid, heparin, and the like.

Cells may be any that can be used for regenerative therapy. Such cells include, for instance, stem cells such as neural stem cells, hematopoietic stem cells, mesenchymal stem cells, liver stem cells, pancreatic stem cells, skin stem cells, muscle stem cells and germ stem cells; blast cells such as myoblasts, osteoblasts, odontoblasts, neuroblastoma, fibroblasts, chondroblasts, retinoblastoma, ameloblasts and cement blasts. Cells may preferably be autologous cells or may be non-autologous cells insofar as they do not induce rejection when applied to the affected sites.

The particulate decellularized tissue of the present invention has the effect of promotion of cellular differentiation and the effect of cellular attraction and is highly effective for cure and regeneration of the affected sites. Thus, the particulate decellularized tissue of the present invention can be used for dermatological usage such as treatment of wound or frostbite of the skin; for surgical usage such as regeneration or replacement of tissues when tissues are removed by surgery or accidents or tissues or organs are damaged by accidents or diseases; for usage in cosmetic surgery such as reconstruction and correction of tissues. For applying the particulate decellularized tissue of the present invention to the affected sites, it may be applied, adhered, sprayed, buried, or injected with a syringe and the like to the affected sites.

The particulate decellularized tissue of the present invention has lesser cytotoxicity and has the effect to promote and induce cellular differentiation. Thus, the particulate decellularized tissue of the present invention can suitably be used as a treatment material for diseases, an auxiliary material for tissue transplant, a scaffold material for regenerative therapy, a base material for cell culture, and the like. When the particulate decellularized tissue of the present invention is used as a treatment material for diseases, an auxiliary material for tissue transplant or a scaffold material for regenerative therapy, it may be applied to the affected portions as it is or after it is processed into gel, sheet, three-dimensional structure, and the like. Alternatively, the particulate decellularized tissue of the present invention may be used for cell culture and then applied to the affected portions.

The present invention is explained in more detail by means of the following Examples but is not limited thereto. Unless otherwise specifically noted, the terms "part" or "%" in Examples refer to mass reference.

### Example 1

Swine liver was frozen and pulverized with a food processer at -20°C and the particles with a diameter of 500 µm or more were removed with a sieve to give fine particles of swine liver (mean particle diameter 50 µm; particles with a particle diameter of less than 1 µm: 5% or less). Five grams of the fine particles and 15 g of saline as a medium for a high hydrostatic pressure treatment were put in a zippered bag made of polyethylene. Using High Pressure Processing Equipment for R&D (Kobe Steel, Ltd.; trade name: Dr. CHEF), a hydrostatic pressure of 1,000 MPa was applied for 15 minutes. Then, the powder obtained after a high hydrostatic pressure treatment was transferred to a sterilized cup. The cup was added with 20 g of a saline as a washing liquid and shaken at 25°C for 5 hours. A washing liquid was replaced with a new one and the cup was shaken for further 2 hours to give a particulate decellularized tissue of Example 1.

### Example 2

The procedures of Example 1 were repeated excepting that swine liver was replaced with swine brain to give a particulate decellularized tissue of Example 2.

### Example 3

The procedures of Example 1 were repeated excepting that swine liver was replaced with swine spinal cord to give a particulate decellularized tissue of Example 3.

### Comparative Example 1

A sterilized cup was added with 5 g of fine particles of swine liver pulverized as in Example 1 and, as a decellularization solution, 15 g of a balanced salt solution of Hanks comprising 0.5% sodium dodecyl sulfate. The cup was stored at 25°C for 5 hours. Then, the decellularization solution was removed and the cup was added with 20 g of a balanced salt solution of Hanks not comprising a surfactant as a washing liquid and was shaken at 25°C for 5 hours. A washing liquid was replaced with a new one and the cup was shaken for further 2 hours to give a particulate decellularized tissue of Comparative Example 1.

### Comparative Example 2

The procedures of Comparative Example 1 were repeated excepting that swine liver was replaced with swine brain to give a particulate decellularized tissue of Comparative Example 2.

### Comparative Example 3

The procedures of Comparative Example 1 were repeated excepting that swine liver was replaced with swine spinal cord to give a particulate decellularized tissue of Comparative Example 3.

### Cell migration test

L929 cells (mouse fibroblasts) were cultured with a MEM medium at 37°C for 24 hours to be starved. To a 24-well plate dish was added 1 mL of MEM medium containing 5% powdered decellularized tissue and a cell culture insert (pore size 8 µm) was set in the respective well. L929 cells cultured in MEM medium were inoculated into the insert (1.0 x 10⁵ cells/well) and cultured at 37°C for 1 to 3 hours and the number of cells which migrated to the well under the insert was measured. The measurement of the number of cells was carried out with a fluorescence microscope after DAPI staining and an average number of 5 wells was calculated. Blank was included without the use of the powdered decellularized tissue. The results are shown in Table 1.

**Table 1**

| | 1 hour later | 2 hours later | 3 hours later |
|---|---|---|---|
| Example 1 | 688 | 1523 | 1520 |
| Comparative Example 1 | 642 | 612 | 628 |
| Blank | 782 | 516 | 505 |

In the cell migration test, the powdered decellularized tissue showed a high cell migration, demonstrating that the powdered decellularized tissue has the effect of cellular attraction. In Comparative Example 1, cell death was- observed in a portion of the cells.

### Neurite outgrowth test

PC12 cells (pheochromocytoma from rat adrenal medulla) were inoculated to a collagen-coated 24-well plate dish with a RPMI medium containing 10% Horse serum and 5% fetal bovine serum (FBS)(1.0 x 10⁴ cells/well) and cultured at 37°C for 24 hours. The culture medium was changed to a RPMI medium containing 0.1% Horse serum and a cell culture insert (pore size 8 µm) was set in the respective well. Into the insert was added 200 µL of saline containing 5% decellularized powder and culture was continued for 24 hours with and without addition of 50 ng of nerve growth factor. The cells were observed with a phase contrast microscope to estimate the effect of neurite outgrowth according to the following criteria. The results are shown in Table 2.
⊚: The effect of neurite outgrowth was apparently observed.
○: The effect of neurite outgrowth was partly observed.
Δ: No effect of neurite outgrowth was observed.
× : Cell death was observed in a portion or all of the cells.

**Table 2**

| | With NGF | Without NGF |
|---|---|---|
| Example 2 | ⊚ | ⊚ |
| Example 3 | ⊚ | ⊚ |
| Comparative Example 2 | ○, × | Δ, × |
| Comparative Example 3 | ○, × | Δ, × |
| Blank | ⊚ | Δ |

In the Neurite outgrowth test, the powdered decellularized tissue apparently showed the effect of neurite outgrowth even without NGF, demonstrating that the powdered decellularized tissue has the effect of induction of cellular differentiation. In Comparative Examples, cell death was observed suggesting that the powdered decellularized tissue of Comparative Examples had cytotoxicity.

### Subcutaneous enthesis test

Rat (Wistar rat, male, 8 to 12 weeks) was anesthetized, shaved on the back, and the shaved portion was disinfected with a povidone iodine solution. The back of the rat was incised about 1.5 cm with scissors to create a subcutaneous pocket, into which about 20 mg of the decellularized powder was embedded. The incised portion was sutured with a suture thread and the sutured portion was again disinfected with a povidone iodine solution. Twenty eight days later, the rat subcutaneous pocket was collected and stained with hematoxylin/eosin stain for histological estimation. Blank was included where the suture was done without embedding of the decellularized powder. The results are shown in Table 3.
○: No inflammatory reaction was observed and cellular attraction was confirmed.
Δ : No inflammatory reaction was observed but cellular attraction was indistinct.
× : Inflammatory reaction was observed and cellular attraction was not confirmed.

### Frostbite model test

Rat (Wistar rat, male, 8 to 12 weeks) was anesthetized, shaved on the back, and the shaved portion was disinfected with a povidone iodine solution. In the back of the rat, deficiency to the mid-dermal layer (diameter 15 mm) was created and thereto a metallic body cooled with liquid nitrogen was pressed for 60 seconds to create wound of frostbite. To the wound was applied the decellularized powder of Example 1 or Comparative Example 2, the wound region was covered with an adhesive plaster, and the whole circumference of the trunk of the rat was covered with a gauze. Seven days later, the wound region was collected and stained with hematoxylin/eosin stain for histological estimation. Blank was included where the wound region was covered with an adhesive plaster without application of the decellularized powder. The results are shown in Table 3.
⊚: Tissue regeneration was observed and no contracture was observed.
○ : Tissue regeneration was observed but contracture was observed to some extent.
Δ : Tissue regeneration was observed but contracture was apparently observed.
×: Deterioration of the affected portion was observed.

**Table 3**

| | Subcutaneous enthesis test | Frostbite model test |
|---|---|---|
| Example 1 | ○ | ⊚ |
| Comparative Example 1 | Δ | × |
| Blank | Δ | Δ |

The subcutaneous enthesis test and the frostbite model test reveal that the powdered decellularized tissue of the present invention is highly effective for regeneration and cure.

### INDUSTRIAL APPLICABILITY

The particulate decellularized tissue of the present invention has lesser cytotoxicity and has the effect of promotion of cellular differentiation and the effect of cellular attraction. Thus, the particulate decellularized tissue of the present invention can suitably be used as a treatment material for diseases, an auxiliary material for tissue transplant, a scaffold material for regenerative therapy, a base material for cell culture, and the like. When the particulate decellularized tissue of the present invention is used as a treatment material for diseases, an auxiliary material for tissue transplant or a scaffold material for regenerative therapy, it may be applied to the affected portions as it is or after it is processed into gel, sheet, three-dimensional structure, and the like. Alternatively, the particulate decellularized tissue of the present invention may be used for cell culture and then applied to the affected portions.

## Claims

1. A particulate decellularized tissue for use in therapy or for use in surgery, wherein said particulate decellularized tissue is prepared by a method for preparing a particulate decellularized tissue comprising a step of applying a high hydrostatic pressure to animal-derived tissues in a medium, wherein said high hydrostatic pressure is 2 to 1,500 MPa, and wherein said high hydrostatic pressure is applied after the animal-derived tissues are pulverized.

2. The particulate decellularized tissue for the use according to claim 1, wherein the method by which the particulate decellularized tissue is prepared comprises that the animal-derived decellularized tissues obtained by applying a high hydrostatic pressure to the animal-derived tissues in a medium are washed with a washing liquid, preferably a washing liquid not comprising an anionic surfactant and/or a nonionic surfactant.

3. The particulate decellularized tissue for the use according to claim 1 or 2, wherein the particulate decellularized tissue is a particulate decellularized tissue for use in treating affected tissue.

4. The particulate decellularized tissue for the use according to claim 3, wherein particulate decellularized tissue is a particulate decellularized tissue for treating a wound or frostbite.

## Patentansprüche

1. Partikelförmig dezellularisiertes Gewebe zur Verwendung bei Therapie oder zur Verwendung bei Operationen, wobei das partikelförmige dezellularisierte Gewebe durch ein Verfahren zur Herstellung eines partikelförmig dezellularisierten Gewebes hergestellt ist, das einen Schritt des Anwendens eines hohen hydrostatischen Drucks auf von einem Tier stammende Gewebe in einem Medium umfasst, wobei der hohe hydrostatische Druck 2 bis 1.500 MPa ist und wobei der hohe hydrostatische Druck angewandt wird, nachdem die vom Tier stammenden Gewebe pulverisiert wurden.

2. Partikelförmig dezellularisiertes Gewebe zur Verwendung nach Anspruch 1, wobei das Verfahren, durch das das partikelförmig dezellularisierte Gewebe hergestellt ist, umfasst, dass die von einem Tier stammenden dezellularisierten Gewebe, die durch Anwendung eines hohen hydrostatischen Drucks auf die von einem Tier stammenden Gewebe in einem Medium erhalten werden, mit einer Waschflüssigkeit gewaschen werden, vorzugsweise einer Waschflüssigkeit, die kein anionisches oberflächenaktives Mittel und/oder nicht-ionisches oberflächenaktives Mittel umfasst.

3. Partikelförmig dezellularisiertes Gewebe zur Verwendung nach Anspruch 1 oder 2, wobei das partikelförmig dezellularisierte Gewebe ein partikelförmig dezellularisiertes Gewebe zur Verwendung bei der Behandlung von betroffenem Gewebe ist.

4. Partikelförmig dezellularisiertes Gewebe zur Verwendung nach Anspruch 3, wobei das partikelförmig dezellularisierte Gewebe ein partikelförmig dezellularisiertes Gewebe für die Behandlung einer Wunde oder einer Erfrierung ist.

## Revendications

1. Tissu décellularisé particulaire pour l'utilisation en thérapie ou pour l'utilisation en chirurgie, ledit tissu décellularisé particulaire étant préparé par une méthode destinée à préparer un tissu décellularisé particulaire, comprenant une étape d'application d'une pression hydrostatique élevée à des tissus dérivés d'animaux dans un milieu, où ladite pression hydrostatique élevée est de 2 à 1500 MPa, et où ladite pression hydrostatique élevée est appliquée après que les tissus dérivés d'animaux sont pulvérisés.

2. Tissu décellularisé particulaire pour l'utilisation selon la revendication 1, ladite méthode par laquelle le tissu décellularisé particulaire est préparé comprenant une étape où les tissus décellularisés dérivés d'animaux obtenus par application d'une pression hydrostatique élevée aux tissus dérivés d'animaux dans un milieu sont lavés avec un liquide de lavage, de préférence un liquide de lavage ne comprenant pas de tensioactif anionique et/ou de tensioactif non ionique.

3. Tissu décellularisé particulaire pour l'utilisation selon la revendication 1 ou 2, ledit tissu décellularisé particulaire étant un tissu décellularisé particulaire pour l'utilisation dans le traitement de tissu affecté.

4. Tissu décellularisé particulaire pour l'utilisation selon la revendication 3, ledit tissu décellularisé particulaire étant un tissu décellularisé particulaire pour le traitement d'une plaie ou d'une gelure.
